# EUROPÄISCHE PATENTANMELDUNG

(11) **EP 2 141 165 A1**
(43) Veröffentlichungstag der Anmeldung: **06.01.2010**
(21) Anmeldenummer: 08159330.3
(22) Anmeldetag: 30.06.2008
(51) Int. Cl.: C07D 457/04, C12P 17/18

(54) **Aufarbeitung von mikrobiologisch hergestellten Mutterkornalkaloiden**

(71) Anmelder: Sandoz AG, 4056 Basel (CH)
(72) Erfinder: Die Erfindernennung liegt noch nicht vor
(74) Vertreter: Englmeier, Ludwig

(57) **Zusammenfassung**

Die vorliegende Erfindung betrifft Verfahren zur Aufarbeitung von mikrobiologisch hergestellten Mutterkornalkaloiden der nachstehend aufgeführten Formel I 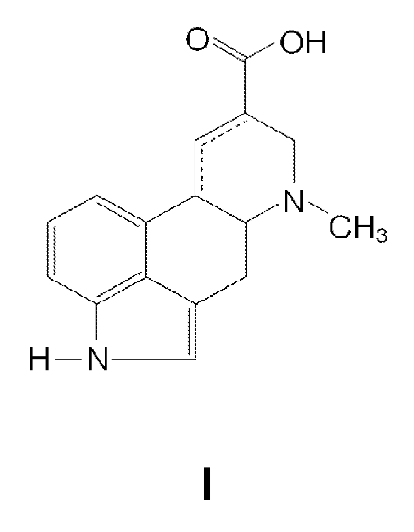 umfassend den Schritt:
a) Extrahieren des bei der biologischen Herstellung anfallenden, wenigstens ein Mutterkornalkaloid der Formel I enthaltenden, Fermentationsprodukts bei einem pH-Wert von 8 - 14 mit einem Extraktionsmittel, das bei 20 °C eine Wasserlöslichkeit von 0,2 g/100 g Wasser bis 25 g/100 g Wasser aufweist, wobei die Menge des Extraktionsmittels ausreicht, zusammen mit dem Fermentationsprodukt ein 2-Phasen-System zu bilden.

## Beschreibung

Die vorliegende Erfindung betrifft Verfahren zur Aufarbeitung von mikrobiologisch hergestellten Mutterkornalkaloiden der nachstehend aufgeführten Formel I 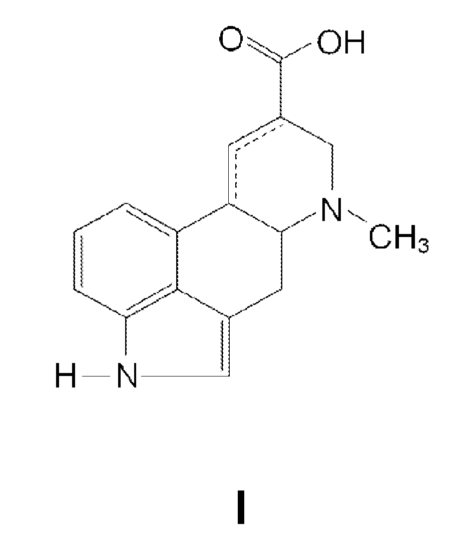 umfassend den Schritt:
a) Extrahieren des bei der biologischen Herstellung anfallenden, wenigstens ein Mutterkornalkaloid der Formel I enthaltenden, Fermentationsprodukts bei einem pH-Wert von 8 - 14 mit einem Extraktionsmittel, das bei 20 °C eine Wasserlöslichkeit von 0,2 g/100 g Wasser bis 25 g/100 g Wasser aufweist, wobei die Menge des Extraktionsmittels ausreicht, zusammen mit dem Fermentationsprodukt ein 2-Phasen-System zu bilden.

Es ist bekannt, dass das Mutterkornalkaloid, Paspalsäure, u. a. eine wichtige Ausgangsverbindung zur Herstellung einer Reihe von Paspalsäurederivaten mit wertvollen pharmakologischen Eigenschaften ist.

Ebenso ist bekannt, dass das Mutterkornalkaloid, Lysergsäure, ein Zwischenprodukt zur Herstellung von wichtigen Arzneimitteln, wie z. B. der Oxitoxica Ergobasin und Methylergobasin oder des Serotoninantagonisten 1-Methyl-d-lysergsäure-(+)-butanolamid-(2') ist. Lysergsäure kann u. a. durch Umlagerung von Paspalsäure gewonnen werden.

Die Herstellung von Paspalsäure oder Lysergsäure mit Hilfe mikrobiologischer Verfahren ist seit langem bekannt und wird großtechnisch ausgeübt. So wurde bereits in der deutschen Offenlegungsschrift DOS 1442294 die Herstellung von Paspalsäure mit Hilfe der Pilzspezies Claviceps paspali bzw. seiner Mutanten [deponiert unter der Nummer NRRL 3080 beim United States Department of Agriculture (Northern Utilization Research and Development Division), Peoria/I11] beschrieben.

Aus der mikrobiologisch hergestellten Paspalsäure kann durch Umlagerung im stark alkalischen Bereich Lysergsäure gewonnen werden. Gemäß der deutschen Offenlegungsschrift DOS 1620375 erfolgt dies vorzugsweise bereits durch Behandlung des Fermentationsfiltrats nach Entfernung der Biomasse mit Basen, wie z. B. verdünnter Ammoniaklösung oder Lösungen von Alkalimetallhydroxiden, ggfs. unter Erwärmung oder erst nach Aufarbeitung und Reinigung der mikrobiologisch hergestellten Paspalsäure im alkalischen Milieu unter Erwärmung. Die Isomerisierung kann auch an einem 2-Phasengemisch unter Einsatz von Tetraalkylammoiumhydroxid, vorzugsweise Tetrabutylammoniumhydroxid, vorgenommen werden (US 6242603).

Die bekannten mikrobiologischen Verfahren zur Herstellung von Mutterkornalkaloiden, insbesondere Paspalsäure bzw. dessen Isomerisierungsprodukt, Lysergsäure, bedingen aufwändige Aufarbeitungsverfahren, wie z. B. die Separierung der Biomasse von dem Fermentationsfiltrat und die damit verbundenen weiteren Reinigungs- bzw. Entfärbungsschritten sowie komplizierte Auftrennschritte des Rohproduktes zum gewünschten Endprodukt. Dies führt nicht nur zu einer aufwändigen und damit längeren Gesamtaufarbeitungszeit, um das gewünschte Stoffwechselprodukt des Fermentationsprozesses zu erhalten, sondern auch zu Ausbeuteverlusten, begrenztem Gesamtdurchsatz des Verfahrens und dadurch zu beachtlichen Herstellungskosten.

Aufgabe der vorliegenden Erfindung war es daher, ein Verfahren zur Aufarbeitung von mikrobiologisch hergestellten Mutterkornalkaloiden, insbesondere von Paspalsäure und Lysergsäure, zur Verfügung zu stellen, das eine Verbesserung hinsichtlich eines oder mehrerer der vorstehend genannten Nachteile des Standes der Technik herbeiführt.

Diese Aufgabe wird durch das zur Verfügung stellen des erfindungsgemäßen Verfahrens zur Aufarbeitung von mikrobiologisch hergestellten Mutterkornalkaloiden, der nachstehend aufgeführten Formel I 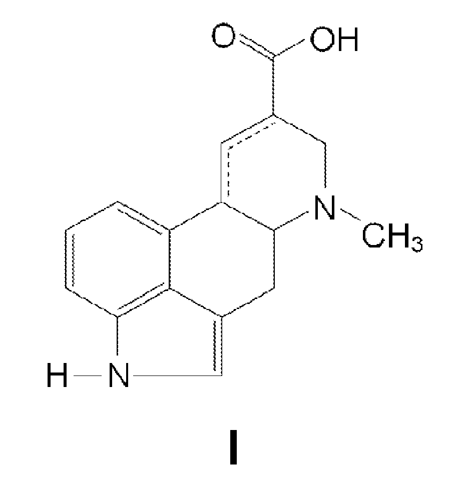 umfassend den Schritt:
a) Extrahieren des bei der biologischen Herstellung anfallenden, wenigstens ein Mutterkornalkaloid der Formel I enthaltenden, Fermentationsprodukts bei einem pH-Wert von 8 - 14 mit einem Extraktionsmittel, das bei 20 °C eine Wasserlöslichkeit von 0,2 g/100 g Wasser bis 25 g/100 g Wasser aufweist, wobei die Menge des Extraktionsmittels ausreicht, zusammen mit dem Fermentationsprodukt ein 2-Phasen-System zu bilden, gelöst.

Überraschenderweise wurde nämlich gefunden, dass Mutterkornalkaloide der Formel I bei einem alkalischen pH-Wert, wie von 8 bis 14, mit einem mäßig polaren Lösungsmittel extrahierbar sind. Erfindungsgemäß wird unter einem mäßig polaren Lösungsmittel ein Lösungsmittel bzw. Extraktionsmittel verstanden, das hinreichend apolar ist, um mit Wasser noch 2-Phasen-Systeme zu bilden, jedoch ausreichend polar, um die bei alkalischem pH-Wert negativ geladenen Mutterkornalkaloide zu lösen. Unerwartet gelingt es nämlich so, die bei alkalischem pH-Wert negativ geladenen Mutterkornalkaloide im Extraktionssystem aus dem wässrigen Fermentationsprodukt und dem erfindungsgemäßen Extraktionsmittel in die Phase des im Vergleich zum wässrigen Fermentationsprodukt weniger polaren Extraktionsmittels überzuführen, was so wegen der Ladung nicht vorhergesehen werden konnte.

Wie bereits vorstehend ausgeführt, werden vorzugsweise zur mikrobiologischen Herstellung der Mutterkornalkaloide, Paspalsäure der Formel I a 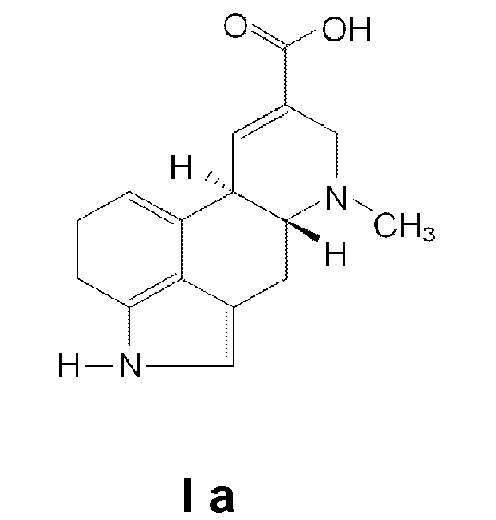 und Lysergsäure der Formel I b 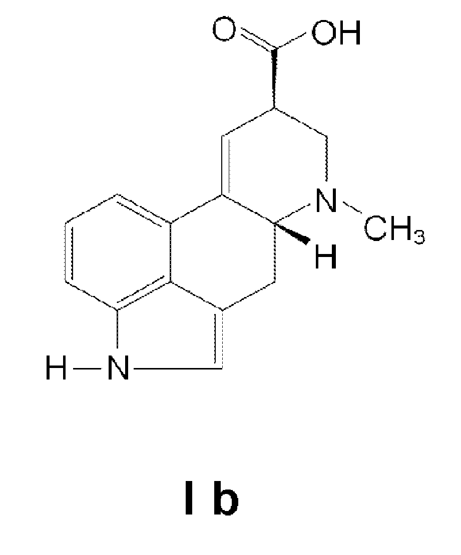 ein spezieller Stamm der Spezies Claviceps paspali Stevens et Hall verwendet. Die Proben dieses Stammes sind beim United States Department of Agriculture (Northern Utilization Research and Development Division), Peoria/I11 unter der Nummer NRRL 3080 deponiert. Die entsprechenden Kulturbedingungen sind u. a. in der deutschen Offenlegungsschrift DOS 1442294, Seiten 5 - 11 offenbart. Die entsprechende Offenbarung wird hiermit als Referenz eingeführt und soll als Teil der Offenbarung der vorliegenden Anmeldung gelten.

Zur erfindungsgemäßen Aufarbeitung der bei dem mikrobiologischen Verfahren erhaltenen Mutterkornalkaloiden, insbesondere der Paspalsäure, kann als Fermentationsprodukt der Gesamt-Fermentationsbrei ohne Abtrennung der Biomasse von dem Fermentationsfiltrat zur weiteren Aufarbeitung verwendet werden. Dieser Gesamt-Fermentationsbrei enthält als Mutterkornalkaloide im wesentlichen Paspalsäure als Hauptstoffwechselprodukt des mikrobiologischen Verfahrens neben geringeren Mengen Lysergsäure und ggfs. Lysergsäurederivaten. Üblicherweise weist der Gesamt-Fermentationsbrei einen Mutterkornalkaloid-Titer von zwischen 0,5 - 40 g/kg Gesamt-Fermentationsbrei auf. Als Fermentationsprodukt, das zur Extraktion verwendet wird, kann auch das Fermentationsfiltrat eingesetzt werden, sofern man den zusätzlichen Aufwand für die Abtrennung der Biomasse vom Fermentationsfiltrat nicht scheut. Vorzugsweise wird erfindungsgemäß als Fermentationsprodukt der Gesamt-Fermentationsbrei ohne Trennung der Biomasse vom Filtrat eingesetzt.

Das Fermentationsprodukt wird mit einem Extraktionsmittel, das bei 20 °C eine Wasserlöslichkeit von 0,2 g/100 g Wasser bis 25 g/100 g Wasser aufweist, mindestens einmal extrahiert. Dabei ist ein Extraktionsmittel, das im wesentlichen frei von halogenierten Kohlenwasserstoffen ist, bevorzugt, wobei dies erfindungsgemäß bedeutet, dass im Extraktionsmittel halogenierte Kohlenwasserstoffe mit einem Anteil von weniger als 2 Volumen % bezogen auf das Gesamtvolumen vorkommen, stärker bevorzugt nicht im Extraktionsmittel vorkommen.

Vorzugsweise wird das Fermentationsprodukt mit einem in Wasser höchstens begrenzt löslichen, kurzkettigen, aliphatischen Alkohol oder Ester als Extraktionsmittel mindestens einmal extrahiert. Vorzugsweise erfolgt die Extraktion mehrmals, besonders bevorzugt zweimal, vorzugsweise mit demselben Extraktionsmittel, wobei die Menge des Extraktionsmittels jeweils ausreicht, zusammen mit dem Fermentationsprodukt ein 2-Phasen-System zu bilden.

Als Extraktionsmittel werden vorzugsweise flüssige, aliphatische Alkohole oder Ester mit einer Wasserlöslichkeit von höchstens 25 g/100 g Wasser (bei 20 °C), vorzugsweise mit einer Wasserlöslichkeit von 0,2 g/100 g Wasser bis 25 g/100 g Wasser, besonders bevorzugt mit einer Wasserlöslichkeit bei 20 °C von 0,5 g/100 g Wasser bis 15 g/100 g Wasser, ganz besonders bevorzugt 2 g/100 g Wasser bis 10 g/100 g Wasser eingesetzt. Solche in Wasser nur begrenzt löslichen Extraktionsmittel sind vorzugsweise aliphatische Alkohole mit wenigstens 4 C-Atomen, besonders bevorzugt Alkohole mit 4 bis 7 C-Atomen, vorzugsweise n-Butanol, Isobutanol und/oder sec.-Butylalkohol und/oder wenigstens ein aliphatischer Ester mit wenigstens 2 bis 4 C-Atomen, vorzugsweise Essigsäuremethylester, Ameisensäureethylester oder Ameisensäuremethylester. Es kann auch ein Gemisch, aus zwei oder mehreren der vorstehend genannten Alkohole, ein Gemisch aus zwei oder mehreren der vorstehend genannten Ester oder ein Gemisch aus je einem oder mehreren der vorstehend genannten Alkohole mit je einem oder mehreren der vorstehend genannten Ester zur Extraktion eingesetzt werden. Ganz besonders bevorzugt wird Butanol wie n-Butanol und/oder sec.-Butylalkohol und/oder Ameisensäureethylester verwendet.

Durch Einsatz der genannten Extraktionsmittel erfolgt die Extraktion der Mutterkornalkaloide gemäß Formel I, insbesondere der Paspalsäure und der gegebenenfalls vorhandenen Lysergsäure, überraschenderweise sehr selektiv, sofern der pH-Wert des Fermentationsproduktes auf einen alkalischen pH-Wert zwischen 8 - 14, vorzugsweise 9,5 bis 11,5 eingestellt worden ist. Besonders bevorzugt wird der pH-Wert auf einen Wert im Bereich von 10,0 - 11,0 eingestellt. Die Einstellung des pH-Wertes kann durch Zugabe von Basen, wie z. B. Alkalimetallhydroxiden, vorzugsweise Natronlauge oder Kalilauge, durch Einleiten von Basen, wie z. B. Ammoniak oder Tetra(C1 - C6)alkylammoniumhydroxid, vorzusweise Tetrabutylammoniumhydroxid, gegebenenfalls in Mischung mit einer untergeordneten Menge an Alkalimetallhydroxiden erfolgen. Vorzugsweise wird während der jeweiligen Extraktion das Fermentationsprodukt zusammen mit dem Extraktionsmittel bewegt, wie z. B. geschüttelt oder gerührt. Es ist auch möglich den Extraktionsschritt im Gegenstromverfahren durchzuführen, wodurch eine zumindest teilweise kontinuierliche Aufarbeitung möglich ist.

Die geeignete Menge Extraktionsmittel, die auch je nach Durchführung der Extraktion schwanken kann, kann durch einfache Vorversuche festgelegt werden und ist nicht begrenzt, solange ausreichend Extraktionsmittel verwendet wird, um mit dem Fermentationsprodukt ein 2-Phasen-System zu bilden. Im Regelfall können 0,2 bis 5 Volumen Extraktionsmittel pro Volumen Fermentationsprodukt mit guten Ergebnissen verwendet werden.

Die entsprechenden Vorrichtungen sind dem Fachmann bekannt, ebenso wie die zum Einsatz kommenden Extraktionsapparaturen. Die durch die Extraktion gewonnenen Extrakte werden typischerweise vereinigt.

Die Temperatur für die Extraktion des Fermentationsproduktes ist ebenfalls nicht besonders begrenzt und kann bei Raumtemperaturen in einem Bereich von 20 - 25 °C erfolgen.

Das gewonnene Extrakt kann zur weiteren Isolierung der Mutterkornalkaloide der Formel I aufkonzentriert werden, indem das Extraktionsmittel zumindest teilweise entfernt wird. Dadurch kann eine nachfolgende Kristallisation des Mutterkornalkaloids erleichtert werden. Dazu wird vorzugsweise mindestens soviel Extraktionsmittel entfernt, dass sich die Konzentration von Mutterkornalkaloiden der Formel I im Konzentrat verdoppelt, vorzugsweise bis auf das 4-Fache erhöht wird. Die Aufkonzentration erfolgt bevorzugt unter schonenden Bedingungen, also zum Beispiel bei Raumtemperatur unter Vakuum. Eine mäßige Temperaturerhöhung, um die Aufkonzentration zu erleichtern, bis zu 70°C zum Beispiel, ist jedoch durchaus möglich, insbesondere bei der Herstellung von Lysergsäure.

In der weiteren Aufarbeitung können dann die Schritte b) und c) erfolgen.

So wird im Schritt b) dem vom übrigen Gesamtfermentationsbei abgetrennten Extrakt eine wässrige Lösung zugegeben, um ein 2-Phasengemisch zu erhalten, und im Schritt c) der pH-Wert des in b) erhaltenen 2-Phasengemisches auf einen Wert von 2,0 bis 8,0, vorzugsweise auf einen Wert von 4,0 bis 6,5 und stärker bevorzugt auf einen Wert, der im wesentlichen dem isoelektrischen Punkt eines der Mutterkornalkaloide gemäß Formel I entspricht, d.h. etwa 5,6 für Paspalsäure und Lysergsäure, eingestellt.

Dadurch geht bzw. gehen die Mutterkornalkaloide gemäß Formel I aus der organischen Phase in die wässrige Phase über. Die Einstellung des sauren pH-Wertes erfolgt vorzugsweise durch Zugabe von Schwefelsäure, Salzsäure oder Eisessig in geeigneter Menge.

Aus dem so erhaltenen 2-Phasengemisch kristallisiert rohes Mutterkornalkaloid der Formel I aus der wässrigen Phase aus, wobei die Kristallisationsgeschwindigkeit durch Abkühlen des 2-Phasengemisches auf Temperaturen bis zu 10 °C beschleunigt werden kann.

Die rohen Mutterkornalkaloide-Kristalle können, z. B. durch einfach Zentrifugation, aus dem 2-Phasengemisch gewonnen werden, ohne dass vorher die wässrige Phase, in die die Mutterkornalkaloide der Formel I übergegangen sind, isoliert werden müssen. Natürlich kann nach dem Übergang der Mutterkornalkaloide in die wässrige Phase diese abgetrennt werden und die rohen Mutterkornalkaloid-Kristalle daraus gewonnen werden, sofern der zusätzliche Aufwand für die Phasentrennung gewünscht ist.

Sofern während des erfindungsgemäßen Aufarbeitungsverfahrens keine Isomerisierung der Paspalsäure in Lysergsäure vorgenommen wird, ist das Hauptprodukt des erfindungsgemäßen Aufarbeitungsverfahrens Paspalsäure, wobei Paspalsäure erfindungsgemäß als ein Gemisch enthaltend Paspalsäure und Lysergsäure, wobei der molare Anteil an Paspalsäure gegenüber der Lysergsäure überwiegt, verstanden wird. So erhält man überaschenderweise bereits nach dem ersten Auskristallisieren Paspalsäure mit einer hohen Gesamtausbeute, vorzugsweise mit mehr als 65 %, wobei die Paspalsäurekristalle bereits eine überraschend hohe Reinheit, vorzugsweise von mehr als 90 %, aufweisen.

Zur weiteren Verbesserung der Reinheit der Paspalsäure und gegebenenfalls zur Erzielung einer vollständigen Entfärbung des Produktes können die so isolierten Paspalsäurekristalle nochmals in geeigneten Lösungsmitteln aufgelöst und gegebenenfalls mit Aktivkohle behandelt werden, um die vollständige Entfärbung zu erzielen und um anschließend durch neuerliches Ansäuern der produkthaltigen Lösung, vorzugsweise auf den isoelektrischen Punkt der Paspalsäure von pH 5,6, diese aus der wässrigen, sauren Lösung auszukristallisieren und dann vorzugsweise nach einer Reinigungswäsche mit wässriger Methanollösung die Paspalsäure-Kristalle bei üblichen Temperaturen zu trocknen. Damit wird erreicht, dass die Reinheit der so gewonnenen Paspalsäurekristalle auf mehr als 95 % bei einer praktisch unveränderten Ausbeute an Paspalsäure von über 65 % steigt.

Sofern mit Hilfe des erfindungsgemäßen Aufarbeitungsverfahrens Lysergsäure als Hauptprodukt erhalten werden soll, umfasst das Verfahren zusätzlich einen weiteren Schritt, bei dem man den pH-Wert einer paspalsäurehaltigen Lösung oder Suspension auf einen Wert ≥ 11 bis 14 einstellt und gegebenenfalls unter Erwärmung die Paspalsäure zu Lysergsäure isomerisiert (umlagert), wobei Lysergsäure erfindungsgemäß als ein Gemisch enthaltend Paspalsäure und Lysergsäure, wobei der molare Anteil an Lysergsäure gegenüber der Paspalsäure überwiegt, verstanden wird. Bevorzugt ist erfindungsgemäß unter Lysergsäure ein Gemisch enthaltend Paspalsäure und Lysergsäure, wobei das molare Verhältnis von Lysergsäure zu Paspalsäure >4 ist, zu verstehen.

Für die Umlagerung bestehen prinzipiell mindestens drei bevorzugte Möglichkeiten, aus den mikrobiologisch hergestellten Mutterkornalkaloiden der Formel I Lysergsäure durch Isomerisierung (Umlagerung) als Hauptprodukt zu erhalten:
1. So ist es prinzipiell möglich, den Gesamt-Fermentationsbrei vor der vorstehend genannten Schritt a), der Extraktion auf vorzugsweise einem pH-Wert von 11 bis 13 einzustellen. Diese Einstellung des pH-Wertes kann vorzugsweise durch Einleiten von Ammoniak, Zugabe von Alkalimetallhydroxiden, vorzugsweise Natronlauge oder Kalilauge, und/oder Tetraalkylammoniumhydroxid, vorzugsweise Tetra(C1-C6)alkylammoniumhydroxid, besonders bevorzugt Tetrabutylammoniumhydroxid, durchgeführt werden.
   Eine Erhöhung der Reaktionstemperatur, vorzugsweise auf 30 - 70 °C, besonders bevorzugt auf 50°C - 70°C ermöglicht, die Dauer der Isomerisierung abzukürzen.
   Sofern eine Erhöhung der Reaktionstemperatur durchgeführt wird, ist es sinnvoll, vor dem Schritt a), der Extraktion des Fermentationsproduktes, vorzugsweise des Gesamt-Fermentationsbreis, mit mindestens einem der vorstehend genannten Extraktionsmitteln, auf Raumtemperatur, vorzugsweise auf 20 - 25 °C, abzukühlen. Darüber hinaus sollte das Fermentationsprodukt auf einen pH-Wert entsprechend den vorstehenden Angabe für die Extraktion in Schritt a) eingestellt werden.
   Der Verlauf der Umlagerungsreaktion kann durch In-Prozess-Kontrolle mit HPLC anhand der Zunahme von Lysergsäure gemäß Formel 1 b und der Abnahme von Paspalsäure gemäß Formel 1a überwacht werden. Typischerweise sind 4h bei pH 12,5 und 50°C ausreichend, um eine im wesentlichen vollständige Umwandlung zu Lysergsäure gemäß Formel 1 b zu bewirken, wobei unter Lysergsäure gemäß Formel 1 b die reine Lysergsäure zu verstehen ist.
   Die weitere Aufarbeitung der so erhaltenen Lysergsäure entspricht den vorstehend angegebenen Verfahrensschritten umfassend die Schritte a), b) und c) zur Aufarbeitung von Mutterkornalkaloiden der Formel I einschließlich einer Umkristallisierung zur Erhöhung der Reinheit des Endproduktes.
2. Alternativ ist es aber auch möglich, die nach dem erfindungsgemäßen Verfahren isolierte Paspalsäure gegebenenfalls nach dem Umkristallisierungsschritt erst dann in die Lysergsäure umzulagern, indem die Paspalsäurekristalle in einem entsprechenden alkalischen Medium, wie in einer Alkalimetalhydroxidlösung, vorzugsweise einer Natrium- oder Kaliumlauge aufgelöst werden, wobei der pH-Wert der Lösung auf einen Wert von 11 bis 14 einzustellen ist, um gegebenenfalls unter Erwärmung auf 30 - 70 °C die Paspalsäure in Lysergsäure umzulagern.
   Wie bereits vorstehend ausgeführt, kann aus dem alkalischen Milieu durch Ansäuern mit den vorstehend angegebenen Säuren auf einen pH-Wert von 2 bis 8, vorzugsweise auf den isoelektrischen Punkt der Lysergsäure, die Lysergsäure auskristallisiert werden. Die entsprechenden Roh-Kristalle können, wie vorstehend angegeben, auch umkristallisiert und abschließend in üblicher Art und Weise getrocknet werden.
3. Natürlich kann der Schritt der alkalikatalysierten Umlagerung von Paspalsäure zu Lysergsäure auch im Zusammenhang mit anderen Verfahrensschritten in der Abfolge des erfindungsgemäßen Aufarbeitungsverfahrens erfolgen, also z. B. erst in der nach der Extraktion des Fermentationsproduktes erhaltenen Extrakt. Da Lysergsäure sich hinsichtlich seines Löslichkeitsverhaltens nicht entscheidend von der Paspalsäure unterscheidet, kann die weitere Aufarbeitung entsprechend die Schritte b) und c) umfassenden Verfahrensweise erfolgen.

Da die erfindungsgemäß zum Einsatz kommenden Extraktionsmittel des Extraktionsschrittes a) überraschend sehr selektiv sind, ist ein weiterer Gegenstand der Erfindung die Verwendung eines Extraktionsmittels, das bei 20° C eine Wasserlöslichkeit von 0,2 g/100 g Wasser bis 25 g/100 g Wasser aufweist, zur Extraktion von Mutterkornalkaloiden der Formel I aus einer wässrigen Lösung oder Suspension, die Mutterkornalkaloide der Formel I enthält, bei einem pH-Wert von 8 - 14.

Vorzugsweise werden für diese Extraktion die vorstehend genannten Extraktionsmittel, die im Schritt a) des erfindungsgemäßen Aufarbeitungsverfahrens zum Einsatz kommen, verwendet, besonders bevorzugt Alkohole mit 4 bis 7 C-Atomen und/oder aliphatische Ester mit wenigstens 2 bis 4 C-Atomen. Ganz besonders bevorzugt wird Butanol wie n-Butanol und/oder sec.-Butylalkohol und/oder Ameisensäureethylester verwendet.

Diese zum Einsatz kommenden Extraktionsmittel lassen sich trotz Reinigungsverfahrens im aufgearbeiteten Mutterkornalkaloid der Formel I nachweisen, z.B. durch die im experimentellen Teil angegebene "Methode zur Bestimmung von 1-Butanol in Mutterkornalkaloiden der Formel I", die ein Fachmann für andere Extraktionsmittel in geeigneter Weise anpassen kann.

Ein weiterer Gegenstand der vorliegenden Erfindung sind daher die nachstehend aufgeführten Produkte:
Paspalsäure, vorzugsweise Paspalsäure-Kristalle, die einen Gehalt von 2 ppm bis 1000 ppm, vorzugsweise 5 ppm bis 700 ppm jeweils eines Extraktionsmittels, das im Schritt a) eingesetzt wurde, vorzugsweise n-Butanol und/oder sec.-Butylalkohol und/oder Ameisensäureethylester aufweist bzw. aufweisen;
Lysergsäure, vorzugsweise Lysergsäure-Kristalle die einen Gehalt von 2 ppm bis 1000 ppm, vorzugsweise 5 ppm bis 700 ppm, jeweils eines Extraktionsmittels, das im Schritt a) eingesetzt wurde, vorzugsweise n-Butanol und/oder sec.-Butylalkohol und/oder Ameisensäureethylester aufweist bzw. aufweisen.

Bevorzugt ist weiterhin Paspalsäure, vorzugsweise in Form von Kristallen, erhältlich nach einem erfindungsgemäßen Verfahren, wobei die Paspalsäure bzw. deren Kristalle 10 ppm bis 500 ppm jeweils eines Extraktionsmittels, das im Schritt a) eingesetzt wurde, vorzugsweise n-Butanol und/oder sec.-Butylalkohol und/oder Ameisensäureethylester, aufweist bzw. aufweisen.

Ebenso bevorzugt ist weiterhin Lysergsäure, vorzugsweise in Form von Kristallen, erhältlich nach einem erfindungsgemäOen Verfahren, wobei die Lysergsäure bzw. dessen Kristalle 10 ppm bis 500 ppm jeweils eines Extraktionsmittel, das im Schritt a) eingesetzt wurde, vorzugsweise n-Butanol und/oder sec.-Butylalkohol und/oder Ameisensäureethylester, aufweist bzw. aufweisen.

Gegenstand der Erfindung ist auch:
1. Ein Verfahren zur Aufarbeitung von mikrobiologisch hergestellten Mutterkornalkaloiden der Formel I 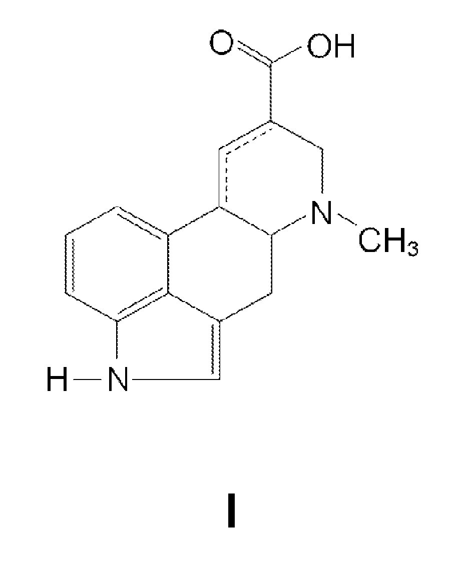 umfassend den Schritt:
   a) Extrahieren des bei der biologischen Herstellung anfallenden, wenigstens ein Mutterkornalkaloid der Formel I enthaltenden, Fermentationsprodukts bei einem pH-Wert von 8 - 14 mit einem Extraktionsmittel, das bei 20 °C eine Wasserlöslichkeit von 0,2 g/100 g Wasser bis 25 g/100 g Wasser aufweist, wobei die Menge des Extraktionsmittels ausreicht, zusammen mit dem Fermentationsprodukt ein 2-Phasen-System zu bilden.
2. Ein Verfahren nach Gegenstand 1, **dadurch gekennzeichnet, dass** das Extraktionsmittel im wesentlichen aus einem Alkohol mit 4 - 7 Kohlenstoffatomen und/oder einem Ester mit 2 - 4 Kohlenstoffatomen besteht.
3. Ein Verfahren nach Gegenstand 1 oder 2, **dadurch gekennzeichnet, dass** das Extraktionsmittel im wesentlichen aus einem Alkohol ausgewählt aus n-Butanol, n-Pentanol, sec.-Butanol und Isobutanol besteht oder im wesentlichen aus einem Ester ausgewählt aus Essigsäuremethylester, Ameisensäuremethylester und Ameisensäureethylester besteht oder im wesentlichen entweder aus einem Gemisch aus zwei oder mehreren der vorstehend genannten Alkohole, einem Gemisch aus zwei oder mehreren der vorstehend genannten Ester oder einem Gemisch aus je einem oder mehreren der vorstehend genannten Alkohole mit je einem oder mehreren der vorstehend genannten Ester besteht.
4. Ein Verfahren nach einem der Gegenstände 1 - 3, **dadurch gekennzeichnet, dass** das Extraktionsmittel bei 20 °C eine Wasserlöslichkeit von 0,5 g/100 g Wasser bis 15 g/100 g Wasser, bevorzugt von 2 g/100 g Wasser bis 10 g/100 g Wasser aufweist.
5. Ein Verfahren nach einem der Gegenstände 1 - 4, **dadurch gekennzeichnet, dass** man den pH-Wert des Fermentationsproduktes vor der Extraktion auf einen Wert im Bereich von 9,5 - 11,5, bevorzugt von 10,0 bis 11,0 einstellt.
6. Ein Verfahren nach einem der Gegenstände 1 - 5, umfassend die weiteren Schritte:
   b) Zugabe einer wässrigen Lösung zu dem vom Fermentationsprodukt abgetrennten Extrakt, um ein 2-Phasengemisch zu erhalten;
   c) Einstellen des pH-Wertes des in b) erhaltenen 2-Phasengemisches auf einen Wert von 2 - 8, um Mutterkornalkaloid der Formel I als Rohprodukt auskristallisieren zu lassen.
7. Ein Verfahren nach einem der Gegenstände 1 - 6, **dadurch gekennzeichnet, dass** man das Rohprodukt der Mutterkornalkaloide der Formel I weiteren Reinigungsschritten zuführt, um für pharmazeutische Zwecke geeignete Mutterkornalkaloide der Formel I zu erhalten.
8. Ein Verfahren nach einem der Gegenstände 1 - 7, **dadurch gekennzeichnet, dass** das Mutterkornalkaloid Paspalsäure ist.
9. Ein Verfahren nach einem der Gegenstände 1 - 7, **dadurch gekennzeichnet, dass** das Mutterkornalkaloid Lysergsäure ist und das Verfahren zusätzlich einen Schritt enthält, bei dem man den ph-Wert einer paspalsäurehaltigen Lösung oder Suspension, auf einen pH-Wert von ≥ 11 bis 14 einstellt und gegebenenfalls unter Erwärmung die Paspalsäure in die Lysergsäure isomerisiert.
10. Ein Verfahren nach Gegenstand 9, **dadurch gekennzeichnet, dass** man den pH-Wert durch Einleiten von Ammoniak, Zugabe von Alkalimetallhydroxiden, vorzugsweise Natronlauge oder Kalilauge, und/oder Tetraalkylammoniumhydroxid, vorzugswese Tetra(C1-C6)alkylammoniumhydroxid auf einen Wert von 11 bis 13 einstellt, um die Isomerisierung von Paspalsäure zu Lysergsäure zu bewirken.
11. Ein Verfahren nach Gegenstand 10, **dadurch gekennzeichnet, dass** man die Isomerisierung bei einer Temperatur zwischen 30 °C und 70°C vornimmt.
12. Ein Verfahren nach einem der Gegenstände 9 - 11, **dadurch gekennzeichnet, dass** die Isomerisierung von Paspalsäure zu Lysergsäure im Fermentationsprodukt vor dem Extraktionsschritt a) stattfindet.
13. Ein Verfahren nach einem der Gegenstände 9 - 11, **dadurch gekennzeichnet, dass** die Isomerisierung von Paspalsäure zu Lysergsäure erfolgt, nachdem man die rohe, kristallisierte Paspalsäure erhalten hat.
14. Verwendung eines Extraktionsmittels, das bei 20° C eine Wasserlöslichkeit von 0,2 g/100 g Wasser bis 25 g/100 g Wasser aufweist, zur Extraktion von Mutterkornalkaloiden der Formel I aus einer wässrigen Lösung oder Suspension, die Mutterkornalkaloide der Formel I enthält, bei einem pH-Wert von 8 - 14.
15. Verwendung nach Gegenstand 14, **dadurch gekennzeichnet, dass** das Extraktionsmittel eine Wasserlöslichkeit von 0,5 g/100 g Wasser bis 15 g/100 g Wasser, bevorzugt 2 g/100 Wasser bis 10 g/100 g Wasser aufweist
16. Verwendung nach Gegenstand 14 oder 15, **dadurch gekennzeichnet, dass** die Mutterkornalkaloide der Formel I Paspalsäure der Formel I a und/oder Lysergsäure der Formel I b sind.
17. Paspalsäure, vorzugsweise Paspalsäure-Kristalle, die einen Gehalt von 2 ppm bis 1000 ppm, vorzugsweise 5 ppm bis 700 ppm eines Extraktionsmittels ausgewählt aus n-Butanol , sec.-Butylalkohol und Ameisensäureethylester, aufweist.
18. Paspalsäure nach Gegenstand 17 erhältlich nach einem Verfahren gemäß einem der Ansprüche 1 - 10.
19. Lysergsäure, vorzugsweise Lysergsäure-Kristalle, die einen Gehalt von 2 ppm bis 1000 ppm, vorzugsweise 5 ppm bis 700 ppm eines Extraktionsmittels ausgewählt aus n-Butanol, sec.-Butylalkohol und Ameisensäureethylester aufweist.
20. Lysergsäure nach Gegenstand 19 erhältlich nach einem Verfahren gemäß einem der Gegenstände 9-13.

### Beispiel 1

1,25 kg Gesamt-Fermentationsbrei, der einen Titer von 7,98 g Mutterkornalkaloid der Formel I / kg hatte, wurde durch Zugabe von NaOH auf einem pH-Wert von 10 eingestellt und bei diesem pH-Wert zweimal mit je 1,25 I n-Butanol extrahiert. Die Ausbeute an Mutterkornalkaloid der Formel I nach den beiden Extraktionsschritten betrug 83 %. Die vereinigten Extrakte wurden auf etwa 30 g Mutterkornalkaloid der Formel I / kg Extrakt konzentriert und mit etwa der gleichen Menge Wasser versetzt. Danach wurde der pH-Wert durch Zugabe von Schwefelsäure auf einen Wert von 5,6 eingestellt. Paspalsäure gelangte von der organischen in die wässrige Phase und kristallisierte aus. Man erhielt so 7,2 g Paspalsäure mit einer Reinheit von 93 %. Das entspricht einer Gesamtausbeute von 67 %.

### Beispiel 2

878 g eines Fermentationsbreis, in dem 2,1 g/kg Lysergsäure und 8,3 g/kg Paspalsäure enthalten waren, wurden 4 Stunden bei pH 12,5 und 50°C gehalten. Nach 4 Stunden enthielt der Brei 7,96 g/kg Lyserg- und 0,37 g/kg Paspalsäure. Der Wirkstoff wurde daraufhin zweimal mit je 900 ml n-BuOH aus dem Brei extrahiert und im Vakuum auf einen Gehalt von 25g/kg aufkonzentriert. Danach wurde der pH-Wert durch Zugabe von Schwefelsäure auf einen Wert von 5,6 eingestellt, wodurch die Lysergsäure auskristallisierte.
Das auf diese Weise erhaltene Rohprodukt hatte eine Reinheit von 75%, die Ausbeute einschließlich der Umsetzung lag bei 50%. Indem beim nächsten Zyklus die Butylacetat Mutterlauge vor der nächsten Umsetzung dem Brei zugegeben wurde, konnte die Ausbeute auf 70% erhöht werden.

### Vergleichsprozess

Dem Paspal- und Lysergsäure enthaltendem Erntebrei wird Aktivkohle zugesetzt. Paspal- und Lysergsäure binden bei neutralen pH-Wert an den Brei bzw. an die Aktivkohle. Die festen Bestandteile werden abzentrifugiert und der Überstand verworfen. Die auf diese Weise isolierte Biomasse samt Aktivkohle wird daraufhin mit einer ammoniakalischen Alkohollösung aufgeschlämmt, wobei sich die beiden Säuren von den Feststoffen lösen. So können die beiden Säuren schließlich nach einer erneuten Zentrifugation von der Biomasse und der Aktivkohle im Überstand gewonnen werden. Wenn man den Überstand aufkonzentriert, fallen Paspal- und Lysergsäure als Rohprodukt aus.

### Methode zur Bestimmung von 1-Butanol in Mutterkornalkaloiden der Formel I

Die Bestimmung von 1-Butanol in Mutterkornalkaloiden der Formel I erfolgte mittels Headspace (HS) Gaschromatographie (GC) Analytik. Dabei wurden ca. 200 mg Mutterkornalkaloidprobe in 20 ml HS-Vials eingewogen und anschließend in 5 ml 1-Methylpyrrolidon aufgenommen.

Die Kalibration erfolgte mit 8 Punkten mit ca. 1, 2, 4, 10, 25, 50, 100, 200 µg 1-Butanol im HS Vial, wobei sehr geringe Mengen 1-Butanol nachweisbar waren.

Der Headspace Modell G1888A von Agilent mit 1 ml Probenschleife wurde mit folgenden Parametern betrieben:

Die Probe wurde 30 min bei 80°C mit der Einstellung "Shaking high" equilibriert. Der Vialdruck von 15 psi wurde 0,2 min eingestellt. Das Füllen der auf 200°C geheizten Probenschleife erfolgte für 0,2 min mit 0,05 min Equilibrierungszeit. Die Injektionszeit in den GC betrug 0,5 min; die Transferline hatte dabei 200°C.

Der Injektorblock war auf 230°C geheizt; das Splitverhältnis betrug 5:1.

Als Säule wurde der Typ DB 624 von J&W mit der Seriennummer US8534615H verwendet. Die Säulendimensionen ware 25 m Länge, 0,2 mm Durchmesser und 1,12 µm Filmdicke der Stationären Phase. Die Säule wurde mit 1 ml konstanter Fluss Helium betrieben. Der GC Ofen wurde mit folgendem Programm betrieben: 40°C wurden für 0,5 min gehalten, dann wurde mit einer Rate von 1°C/min auf 45°C geheizt, dann mit 15°C/min auf 180°C und anschließend mit 30°C/min auf 220°C; diese Temperatur wurde für 4,5 min gehalten.

Als Detektor wurde ein Flammenionisationsdetektor (FID) verwendet. Dieser hatte 250°C und wurde mit 30 ml/min Wasserstoff und 400 ml/min synthetischer Luft betrieben. Das analoge Signal wurde mit der Range 1 an den Chromeleon A/D Wandler übertragen.

## Patentansprüche

1. Ein Verfahren zur Aufarbeitung von mikrobiologisch hergestellten Mutterkornalkaloiden der Formel I 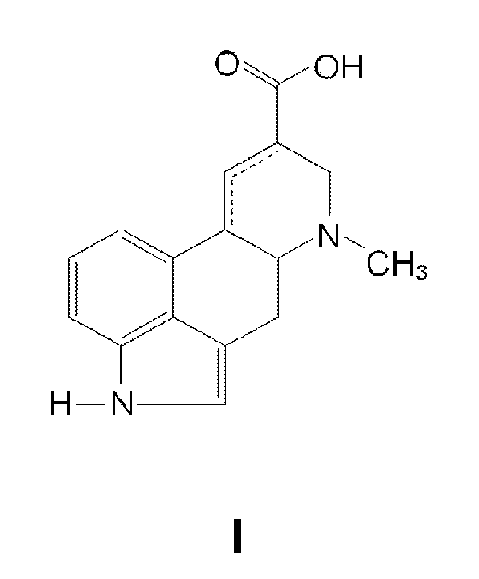 umfassend den Schritt:
a) Extrahieren des bei der biologischen Herstellung anfallenden, wenigstens ein Mutterkornalkaloid der Formel 1 enthaltenden, Fermentationsprodukts bei einem pH-Wert von 8 - 14 mit einem Extraktionsmittel, das bei 20 °C eine Wasserlöslichkeit von 0,2 g/100 g Wasser bis 25 g/100 g Wasser aufweist, wobei die Menge des Extraktionsmittels ausreicht, zusammen mit dem Fermentationsprodukt ein 2-Phasen-System zu bilden.

2. Ein Verfahren nach Anspruch 1, **dadurch gekennzeichnet, dass** das Extraktionsmittel im wesentlichen aus einem Alkohol mit 4 - 7 Kohlenstoffatomen und/oder einem Ester mit 2 - 4 Kohlenstoffatomen besteht.

3. Ein Verfahren nach Anspruch 1 oder 2, **dadurch gekennzeichnet, dass** das Extraktionsmittel im wesentlichen aus einem Alkohol ausgewählt aus n-Butanol, n-Pentanol, sec.-Butanol und Isobutanol besteht oder im wesentlichen aus einem Ester ausgewählt aus Essigsäuremethylester, Ameisensäuremethylester und Ameisensäureethylester besteht oder im wesentlichen entweder aus einem Gemisch aus zwei oder mehreren der vorstehend genannten Alkohole, einem Gemisch aus zwei oder mehreren der vorstehend genannten Ester oder einem Gemisch aus je einem oder mehreren der vorstehend genannten Alkohole mit je einem oder mehreren der vorstehend genannten Ester besteht.

4. Ein Verfahren nach einem der Ansprüche 1 - 3, **dadurch gekennzeichnet, dass** man den pH-Wert des Fermentationsproduktes vor der Extraktion auf einen Wert im Bereich von 9,5 - 11,5, bevorzugt von 10,0 bis 11,0 einstellt.

5. Ein Verfahren nach einem der Ansprüche 1 - 4, umfassend die weiteren Schritte:
b) Zugabe einer wässrigen Lösung zu dem vom Fermentationsprodukt abgetrennten Extrakt, um ein 2-Phasengemisch zu erhalten;
c) Einstellen des pH-Wertes des in b) erhaltenen 2-Phasengemisches auf einen Wert von 2 - 8, um Mutterkornalkaloid der Formel I als Rohprodukt auskristallisieren zu lassen.

6. Ein Verfahren nach einem der Ansprüche 1 - 5, **dadurch gekennzeichnet, dass** das Mutterkornalkaloid Lysergsäure ist und das Verfahren zusätzlich einen Schritt enthält, bei dem man den ph-Wert einer paspalsäurehaltigen Lösung oder Suspension, auf einen pH-Wert von ≥ 11 bis 14 einstellt und gegebenenfalls unter Erwärmung die Paspalsäure in die Lysergsäure isomerisiert.

7. Verwendung eines Extraktionsmittels, das bei 20° C eine Wasserlöslichkeit von 0,2 g/100 g Wasser bis 25 g/100 g Wasser aufweist, zur Extraktion von Mutterkornalkaloiden der Formel I aus einer wässrigen Lösung oder Suspension, die Mutterkornalkaloide der Formel I enthält, bei einem pH-Wert von 8 - 14.

8. Verwendung nach Anspruch 7, **dadurch gekennzeichnet, dass** das Extraktionsmittel eine Wasserlöslichkeit von 0,5 g/100 g Wasser bis 15 g/100 g Wasser, bevorzugt 2 g/100 Wasser bis 10 g/100 g Wasser aufweist

9. Paspalsäure, vorzugsweise Paspalsäure-Kristalle, die einen Gehalt von 2 ppm bis 1000 ppm, vorzugsweise 5 ppm bis 700 ppm eines Extraktionsmittels ausgewählt aus n-Butanol , sec.-Butylalkohol und Ameisensäureethylester, aufweist.

10. Lysergsäure, vorzugsweise Lysergsäure-Kristalle, die einen Gehalt von 2 ppm bis 1000 ppm, vorzugsweise 5 ppm bis 700 ppm eines Extraktionsmittels ausgewählt aus n-Butanol, sec.-Butylalkohol und Ameisensäureethylester aufweist.
